# EUROPEAN PATENT APPLICATION

(11) **EP 4 810 050 A1**
(43) Date of publication of application: **23.09.2026**
(21) Application number: 23958869.2
(22) Date of filing: 16.11.2023
(51) Int. Cl.: A61K 35/74, A61K 9/48, A61K 47/32, A61K 47/36, A61K 47/38

(54) **PREPARATION COMPOSITION**

(71) Applicant: Miyarisan Pharmaceutical Co., Ltd., Sakaki-machi Hanishina-gun Nagano 389-0682 (JP)
(72) Inventor: OKA, Kentaro, Tokyo 114-0016 (JP); KOBAYASHI, Noriko, Tokyo 114-0016 (JP); HORIUCHI, Hiroto, Tokyo 114-0016 (JP); KOBAYASHI, Yuya, Tokyo 114-0016 (JP)
(74) Representative: J A Kemp LLP
(86) International application number: PCT/JP2023/041230
(87) International publication number: WO 2025/104863

(57) **Abstract**

Provided is a new formulation composition capable of improving disintegratability of a solid formulation. A formulation composition containing: a bacterial cell; at least one disintegrant selected from the group consisting of croscarmellose sodium, crospovidone, and sodium starch glycolate; and an excipient (provided that, croscarmellose sodium, crospovidone, and sodium starch glycolate are excluded), in which a content of the bacterial cell is 3.0 mass% or more and 35.0 mass% or less, when the content of the bacterial cell is 3.0 mass% or more and 29.5 mass% or less, a content of the disintegrant is 5.0 mass% or more and 14.0 mass% or less, and when the content of the bacterial cell is more than 29.5 mass% and 35.0 mass% or less, the content of the disintegrant is 10.0 mass% or more and 14.0 mass% or less

## Description

### TECHNICAL FIELD

The present invention relates to a formulation composition.

### BACKGROUND ART

One form of formulation is a solid formulation. The solid formulation is a commonly used dosage form, and refers to a solid-state formulation such as an encapsulated formulation or a tablet. The solid formulation contains an additive such as an excipient in addition to a medicinal ingredient, an active ingredient, and the like.

Microorganisms are attracting attention as one of a medicinal ingredient, an active ingredient, and the like of formulations. As such microorganisms, probiotics that are "viable microorganisms that beneficially influence host health by improving intestinal flora" are used.

In a solid formulation containing microorganisms, the content of microorganisms in the solid formulation is changed according to circumstances. However, when the content of microorganisms is increased, disintegratability of the solid formulation may be deteriorated.

In order to solve such a problem, JP 2022-031035 A discloses a tablet containing lactic acid bacteria powder, maltose powder, and a binder at a specific ratio.

### SUMMARY OF INVENTION

An object of the present invention is to provide a new formulation composition capable of improving disintegratability of a solid formulation.

The present inventors have surprisingly found that disintegratability of a solid formulation can be improved by setting the content of a specific disintegrant with respect to the content of a bacterial cell (microorganisms) to a predetermined range. Based on this finding, the present inventors have completed the present invention.

That is, according to one embodiment of the present invention, provided is a formulation composition containing: a bacterial cell; at least one disintegrant selected from the group consisting of croscarmellose sodium, crospovidone, and sodium starch glycolate; and an excipient (provided that, croscarmellose sodium, crospovidone, and sodium starch glycolate are excluded), in which a content of the bacterial cell is 3.0 mass% or more and 35.0 mass% or less, when the content of the bacterial cell is 3.0 mass% or more and 29.5 mass% or less, a content of the disintegrant is 5.0 mass% or more and 14.0 mass% or less, and when the content of the bacterial cell is more than 29.5 mass% and 35.0 mass% or less, the content of the disintegrant is 10.0 mass% or more and 14.0 mass% or less.

### BRIEF DESCRIPTION OF DRAWINGS

Fig. 1 is a graph showing the results of a disintegration test of an encapsulated formulation of Comparative Example 1.
Fig. 2 is a graph showing the results of a disintegration test of an encapsulated formulation of Comparative Example 2.
Fig. 3 is a graph showing the results of a disintegration test of encapsulated formulations of Examples 4 and 5 and Comparative Examples 3 to 8.
Fig. 4 is a graph showing the results of a disintegration test of encapsulated formulations of Examples 6 to 8.
Fig. 5 is a graph showing the results of a disintegration test of encapsulated formulations of Examples 9 and 10.
Fig. 6 is a graph showing the measurement results of filling amounts of encapsulated formulations of Examples 3 and 11.

### DESCRIPTION OF EMBODIMENTS

Hereinafter, embodiments according to an aspect of the present invention will be described. The present invention is not limited only to the following embodiments.

In the present specification, the phrase "X to Y" indicating a range means "X or more and Y or less". Unless otherwise specified, operations and measurements of physical properties and the like are measured under the conditions of room temperature (20 to 25°C)/relative humidity of 40 to 50% RH.

### <Formulation composition>

One embodiment of the present invention relates to a formulation composition containing: a bacterial cell; at least one disintegrant selected from the group consisting of croscarmellose sodium, crospovidone, and sodium starch glycolate; and an excipient (provided that, croscarmellose sodium, crospovidone, and sodium starch glycolate are excluded), in which a content of the bacterial cell is 3.0 mass% or more and 35.0 mass% or less, when the content of the bacterial cell is 3.0 mass% or more and 29.5 mass% or less, a content of the disintegrant is 5.0 mass% or more and 14.0 mass% or less, and when the content of the bacterial cell is more than 29.5 mass% and 35.0 mass% or less, the content of the disintegrant is 10.0 mass% or more and 14.0 mass% or less. With such a configuration, disintegratability of a solid formulation can be improved.

In the present specification, "at least one disintegrant selected from the group consisting of croscarmellose sodium, crospovidone, and sodium starch glycolate" is also simply referred to as "disintegrant according to the present invention".

In the present specification, when disintegrants other than "croscarmellose sodium, crospovidone, and sodium starch glycolate" have use as an excipient, these disintegrants are handled as an excipient.

In the present specification, when the formulation composition according to the present invention contains a bacterial cell in the form of bacterial powder, components other than the bacterial cell in the bacterial powder are handled as corresponding components (a disintegrant, an excipient, a lubricant, or other components).

The formulation composition according to the present invention contains a bacterial cell.

The bacterial cell is not particularly limited, and a bacterial cell used in the formulation can be used. The bacterial cell is preferably a bacterial cell of at least one bacteria selected from the group consisting of butyric acid bacteria, lactic acid bacteria, and bifidobacteria.

Examples of the butyric acid bacteria include butyric acid bacteria belonging to the genus Clostridium having no pathogenicity. Specific examples of the butyric acid bacteria include *Clostridium butyricum*. The butyric acid bacteria are known and available by a conventional method.

Specific examples of the *Clostridium butyricum* include *Clostridium butyricum* MIYAIRI 588 (FERM BP-2789), *Clostridium butyricum* NIP1020, *Clostridium butyricum* NIP1021, *Clostridium butyricum* (FERM P-11868), *Clostridium butyricum* (FERM P-11868), *Clostridium butyricum* (FERM P-11869), *Clostridium butyricum* (FERM P-11870), *Clostridium butyricum* ATCC859, *Clostridium butyricum* NBRC3315, *Clostridium butyricum* ATCC860, *Clostridium butyricum* ATCC19398, and the like. All of these are deposited and stored in National Institute of Technology and Evaluation, Patent Microorganisms Depositary. The *Clostridium butyricum* is preferably one or more selected from the group consisting of *Clostridium butyricum* MIYAIRI 588 (FERM BP-2789), *Clostridium butyricum* MIYAIRI 585 (FERM BP-06815), *Clostridium butyricum* MIYAIRI 595 (FERM BP-06816), and *Clostridium butyricum* MIYAIRI 630 (FERM BP-06817), and more preferably *Clostridium butyricum* MIYAIRI 588 (FERM BP-2789). C*lostridium butyricum* MIYAIRI 588 strain was deposited as FERM BP-2789 with Fermentation Research Institute, Agency of Industrial Science and Technology (currently called National Institute of Technology and Evaluation, International Patent Organism Depositary) (Room 120, 2-5-8 Kazusakamatari, Kisarazu, Chiba 292-0818, Japan) on May 1, 1981, and was transferred to the International Depositary Authority under the Budapest Treaty on March 6, 1990 and was deposited as accession number FERM BP-2789.

Examples of the lactic acid bacteria include lactic acid bacteria belonging to the genus *Lactobacillus* and the genus *Enterococcus*, which have no pathogenicity. Specific examples of the lactic acid bacteria include *Lactobacillus aciddophilus*, *Lactobacillus casei*, *Lactobacillus paracasei*, *Lactobacillus plantarum*, *Lactobacillus buigericus*, *Lactobacillus rhamnosus*, *Lactobacillus gasseri*, *Lactobacillus fermentum*, *Lactobacillus curvatus*, *Lactobacillus reuteri*, *Lactobacillus brevis*, *Lactobacillus buchneri*, *Lactobacillus delbrueckii*, *Lactobacillus helveticus*, *Lactobacillus salibarius*, *Lactobacillus farciminis*, *Enterococcus faecalis*, *Enterococcus faecium*, and the like. These lactic acid bacteria are known and available by a conventional method.

Examples of the bifidobacteria include bifidobacteria belonging to the genus *Bifidobacterium* having no pathogenicity. Specific examples of the bifidobacteria include *Bifidobacterium bifidum*, *Bifidobacterium infantis*, *Bifidobacterium longum*, *Bifidobacterium breve*, *Bifidobacterium lactis*, *Bifidobacterium animalis*, and the like. These bifidobacteria are known and available by a conventional method.

In the formulation composition according to the present invention, the content of the bacterial cell is 3.0 mass% or more and 35.0 mass% or less with respect to the total mass of the formulation composition. The content of the bacterial cell may be 4.0 mass% or more and 34.0 mass% or less, 8.0 mass% or more and 34.0 mass% or less, 20.0 mass% or more and 34.0 mass% or less, or 25.0 mass% or more and 34.0 mass% or less, with respect to the total mass of the formulation composition.

In the formulation composition according to the present invention, the bacterial cell is preferably in the form of a dried product (bacterial powder), and more preferably in the form of a dried product prepared by spray drying or a fluidized bed granulation method. A method for forming a bacterial cell into a dried product (preferably, spray drying and a fluidized bed granulation methods) is not particularly limited, and conventionally known methods can be used. In the present specification, the excipient in the dried product of the bacterial cell is handled as an excipient described below. When the dried product of the bacterial cell contains further a disintegrant, a lubricant, and other components, the disintegrant, the lubricant, and the other components are handled as a disintegrant, a lubricant, and other components described below, respectively.

The formulation composition according to the present invention contains at least one disintegrant selected from the group consisting of croscarmellose sodium, crospovidone, and sodium starch glycolate.

In the formulation composition according to the present invention, when the content of the bacterial cell is 3.0 mass% or more and 29.5 mass% or less with respect to the total mass of the formulation composition, the content of the disintegrant is 5.0 mass% or more and 14.0 mass% or less with respect to the total mass of the formulation composition, the content of the bacterial cell is more than 29.5 mass% and 35.0 mass% or less with respect to the total mass of the formulation composition, and the content of the disintegrant is 10.0 mass% or more and 14.0 mass% or less with respect to the total mass of the formulation composition.

In one embodiment,
when the content of the bacterial cell is 4.0 mass% or more and 29.5 mass% or less with respect to the total mass of the formulation composition, the content of the disintegrant is 5.0 mass% or more and 14.0 mass% or less, 5.0 mass% or more and 10.0 mass% or less, or 10.0 mass% or more and 14.0 mass% or less, with respect to the total mass of the formulation composition;
when the content of the bacterial cell is 8.0 mass% or more and 29.5 mass% or less with respect to the total mass of the formulation composition, the content of the disintegrant is 5.0 mass% or more and 14.0 mass% or less, 5.0 mass% or more and 10.0 mass% or less, or 10.0 mass% or more and 14.0 mass% or less, with respect to the total mass of the formulation composition;
when the content of the bacterial cell is 20.0 mass% or more and 29.5 mass% or less with respect to the total mass of the formulation composition, the content of the disintegrant is 5.0 mass% or more and 14.0 mass% or less, 5.0 mass% or more and 10.0 mass% or less, or 10.0 mass% or more and 14.0 mass% or less with respect to the total mass of the formulation composition;
when the content of the bacterial cell is 25.0 mass% or more and 29.5 mass% or less with respect to the total mass of the formulation composition, the content of the disintegrant is 5.0 mass% or more and 14.0 mass% or less, 5.0 mass% or more and 10.0 mass% or less, or 10.0 mass% or more and 14.0 mass% or less, with respect to the total mass of the formulation composition; and
when the content of the bacterial cell is more than 29.5 mass% and 34.0 mass% or less with respect to the total mass of the formulation composition, the content of the disintegrant is 10.0 mass% or more and 14.0 mass% or less with respect to the total mass of the formulation composition.

The formulation composition according to the present invention contains an excipient (provided that, croscarmellose sodium, crospovidone, and sodium starch glycolate are excluded).

In the formulation composition according to the present invention, the content of the excipient can be appropriately adjusted according to the contents of the bacterial cell and the disintegrant. The content of the excipient is preferably 50.0 mass% or more and 85.0 mass% or less with respect to the total mass of the formulation composition.

The excipient is not particularly limited as long as it is an excipient used for pharmaceuticals. Examples of the excipient include sugars and sugar alcohols such as lactose (lactose hydrate), saccharose, sucrose, glucose, starch syrup, cellulose, and mannitol; starches such as wheat starch, potato starch, and corn starch; dextrins such as cyclodextrin; cellulose derivatives such as carboxymethyl cellulose (CMC), hydroxyethyl cellulose, hydroxypropyl cellulose, low-substituted hydroxypropyl cellulose, and hydroxypropyl methyl cellulose; substituted polymer compounds such as polyvinyl alcohol and polyvinyl pyrrolidone; minerals (components) such as talc and light anhydrous silicic acid; inorganic salts such as precipitated calcium carbonate; and the like.

In a preferred embodiment, the formulation composition according to the present invention contains at least one of lactose hydrate and mannitol, and corn starch as excipients.

When the excipient contains at least one of lactose hydrate and mannitol, and corn starch, the content of the at least one of lactose hydrate and mannitol is preferably 12.0 mass% or more and 47.0 mass% or less with respect to the total mass of the excipient. The content of the corn starch is preferably 47.0 mass% or more 78.0 mass% or less with respect to the total mass of the excipient. The mass ratio of the corn starch to the at least one of lactose hydrate and mannitol is preferably 1.0 or more and 6.4 or less.

In a preferred embodiment, the formulation composition according to the present invention contains crystalline cellulose as an excipient, in addition to at least one of lactose hydrate and mannitol, and corn starch.

When the excipient further contains crystalline cellulose in addition to at least one of lactose hydrate and mannitol, and corn starch, the content of the crystalline cellulose is preferably 5.9 mass% or more and 9.8 mass% or less with respect to the total mass of the excipient. The mass ratio of the crystalline cellulose to the at least one of lactose hydrate and mannitol is preferably 0.12 or more and 0.80 or less.

When the excipient contains at least one of lactose hydrate and mannitol, corn starch, and crystalline cellulose, the content of the at least one of lactose hydrate and mannitol, corn starch, and crystalline cellulose is preferably 95 mass% or more and 100 mass% or less, more preferably 98 mass% or more and 100 mass% or less, and still more preferably 100 mass%, with respect to the total mass of the excipient.

The formulation composition according to the present invention can further contain a lubricant.

Examples of the lubricant include stearates (for example, magnesium stearate), refined talc, borax, polyethylene glycol, and the like.

In the formulation composition according to the present invention, the content of the lubricant is, for example, 0.1 mass% or more and 2 mass% or less and preferably 0.5 mass% or more and 1.5 mass% or less with respect to the total mass of the formulation composition.

The formulation composition according to the present invention can contain other components in addition to the above-described components. Examples of the other components include an additive, a pharmaceutically acceptable carrier, an auxiliary component, and the like.

Examples of the additive include a stabilizer, a preservative, a wetting agent, an emulsifier, a sweetener, a coloring agent, a fragrance, a buffering agent, an antioxidant, a pH adjusting agent, a binder, a thickener, a dispersant, a suspending agent, a bacteriostat, a surfactant, a disintegrant (excluding croscarmellose sodium, crospovidone, and sodium starch glycolate), and the like.

Examples of the pharmaceutically acceptable carrier include binders such as dextrin and cellulose; solvents such as water and organic solvents; and the like.

Examples of the auxiliary component include antibiotics, vitamins (for example, vitamin C and vitamin E), amino acids, peptides, minerals (for example, zinc, iron, copper, manganese, and the like), nucleic acids, polysaccharides, fatty acids, galenical pharmaceuticals, and the like.

A method for producing the formulation composition according to the present invention is not particularly limited, and examples thereof include a method in which the bacterial cell, the disintegrant according to the present invention, the excipient, and as necessary, a lubricant and other components are mixed, and the like.

Since the formulation composition according to the present invention is excellent in disintegratability, the formulation composition is preferably used in the form of a solid formulation (oral solid formulation). Examples of the solid formulation include encapsulated formulations, tablets, powders, fine granules, granules, pills, sustained preparations, and the like. Since the formulation composition according to the present invention can suppress variation in the filling amount in addition to disintegratability, the formulation composition is preferably in the form of an encapsulated formulation. Therefore, one embodiment of the present invention relates to an encapsulated formulation containing the formulation composition according to the present invention.

A method for producing the solid formulation is not particularly limited, and conventionally known methods can be used. For example, an encapsulated formulation can be produced by filling a capsule with the formulation composition according to the present invention using a capsule filling machine. As the capsule, a known capsule can be used. Examples of the capsule include hydroxypropyl methylcellulose (HPMC) capsules, gelatin capsules, and the like. The size of the capsule is not particularly limited, and can be appropriately selected according to the amount of the formulation composition to be filled. The capsule filling machine is not particularly limited, and a fully automatic capsule filling machine, a tabletop capsule filling machine, or the like can be used. Since the formulation composition according to the present invention can suppress variation in the filling amount, a fully automatic capsule filling machine is preferably used.

### <Embodiments>

Embodiments of the present invention are illustrated below.
[1] A formulation composition containing:
   a bacterial cell;
   at least one disintegrant selected from the group consisting of croscarmellose sodium, crospovidone, and sodium starch glycolate; and
   an excipient (provided that, croscarmellose sodium, crospovidone, and sodium starch glycolate are excluded), wherein
   a content of the bacterial cell is 3.0 mass% or more and 35.0 mass% or less with respect to a total mass of the formulation composition,
   when the content of the bacterial cell is 3.0 mass% or more and 29.5 mass% or less with respect to the total mass of the formulation composition, a content of the disintegrant is 5.0 mass% or more and 14.0 mass% or less with respect to the total mass of the formulation composition, and
   when the content of the bacterial cell is more than 29.5 mass% and 35.0 mass% or less with respect to the total mass of the formulation composition, the content of the disintegrant is 10.0 mass% or more and 14.0 mass% or less with respect to the total mass of the formulation composition.
[2] The formulation composition according to [1], wherein the bacterial cell is a bacterial cell of at least one bacteria selected from the group consisting of butyric acid bacteria, lactic acid bacteria, and bifidobacteria.
[3] The formulation composition according to [1] or [2], wherein the bacterial cell is in a form of a dried product prepared by spray drying or a fluidized bed granulation method.
[4] The formulation composition according to any one of [1] to [3], wherein a content of the excipient is 50.0 mass% or more and 85.0 mass% or less with respect to the total mass of the formulation composition.
[5] The formulation composition according to any one of [1] to [4], wherein the excipient contains at least one of lactose hydrate and mannitol, and corn starch.
[6] The formulation composition according to [5], wherein a content of the at least one of lactose hydrate and mannitol is 12.0 mass% or more and 47.0 mass% or less with respect to the total mass of the excipient.
[7] The formulation composition according to [5] or [6], wherein a mass ratio of the corn starch to the at least one of lactose hydrate and mannitol is 1.0 or more and 6.4 or less.
[8] The formulation composition according to any one of [5] to [7], wherein the excipient further contains crystalline cellulose.
[9] The formulation composition according to [8], wherein a mass ratio of the crystalline cellulose to the at least one of lactose hydrate and mannitol is 0.12 or more and 0.80 or less.
[10] The formulation composition according to any one of [1] to [9], further comprising a lubricant.
[11] An encapsulated formulation containing the formulation composition according to any one of [1] to [10].

### EXAMPLES

Hereinafter, the present invention will be described using specific examples, but the present invention is not limited to these examples. Unless otherwise specified, the operation was performed at room temperature (25°C).

### <Materials>

### (Bacterial powder)

- Bacterial powder of *Clostridium butyricum* MIYAIRI 588 (FERM BP-2789) (preparation method: spray drying method, content of bacterial cell: 42.1 mass%, content of excipient: 57.9 mass%)

### (Excipient)

- Lactose hydrate (Sheffield^{™} Spray Dried 315, KERRYGROUP P.L.C., Tralee)
- Corn starch (Corn Starch White W-4P, JAPAN CORN STARCH CO., LTD.)
- Crystalline cellulose (Ceolus PH-101, Asahi Kasei Corp.)

### (Disintegrant)

- Croscarmellose sodium (KICCOLATE ND-2HS, Asahi Kasei Corp.)
- Crospovidone (Kollidon CL, BASF Japan Ltd.)
- Sodium starch glycolate (Primojel, DFE Pharma GmbH&Co.KG, Goch)

### (Lubricant)

- Magnesium stearate (Magnesium stearate-S, NOF CORPORATION)

### (Capsule)

- Hypromellose capsule No. 3 (HPMC capsule QUALI-V for medical use, Qualicaps Co., Ltd.).

### <Preparation of encapsulated formulation>

Each material was added to a bag so as to be mass% shown in Table 1, and the mixture was inverted and mixed for 2 minutes to prepare a formulation composition. The formulation composition and hypromellose capsule were added to a capsule filling machine (F-40, Qualicaps Co., Ltd.) to prepare an encapsulated formulation (about 246 mg (contents: 200 mg), length: about 15.8 mm). The operation speed was set to 40,000 cap/h.

**[Table 1-1]**

| (Table 1) | | | | | | | | | |
|---|---|---|---|---|---|---|---|---|---|
| | Comparative Example 1 | Comparative Example 2 | Example 1 | Example 2 | Example 3 | Comparative Example 3 | Comparative Example 4 | Comparative Example 5 | Comparative Example 6 |
| Bacterial cell (mass%) | 26.6 | 33.7 | 25.3 | 29.5 | 33.7 | 0.8 | 0.8 | 2.1 | 2.1 |
| Lactose hydrate (mass%) | 24.9 | 20.3 | 18.7 | 14.5 | 10.3 | 43.2 | 53.2 | 41.9 | 51.9 |
| Corn starch (mass%) | 39.9 | 40.0 | 40.0 | 40.0 | 40.0 | 40.0 | 40.0 | 40.0 | 40.0 |
| Crystalline cellulose (mass%) | 7.6 | 5.0 | 5.0 | 5.0 | 5.0 | 5.0 | 5.0 | 5.0 | 5.0 |
| Croscarmellose Na (mass%) | 0.0 | 0.0 | 10.0 | 10.0 | 10.0 | 10.0 | 0.0 | 10.0 | 0.0 |
| Crospovidone (mass%) | 0.0 | 0.0 | 0.0 | 0.0 | 0.0 | 0.0 | 0.0 | 0.0 | 0.0 |
| Na starch glycolate (mass%) | 0.0 | 0.0 | 0.0 | 0.0 | 0.0 | 0.0 | 0.0 | 0.0 | 0.0 |
| Magnesium stearate (mass%) | 1.0 | 1.0 | 1.0 | 1.0 | 1.0 | 1.0 | 1.0 | 1.0 | 1.0 |
| Total | 100.0 | 100.0 | 100.0 | 100.0 | 100.0 | 100.0 | 100.0 | 100.0 | 100.0 |
| Lactose hydrate/entire excipient (mass%) | 34.4 | 31.1 | 29.4 | 24.4 | 18.7 | 49.0 | 54.2 | 48.2 | 53.6 |
| Corn starch/entire excipient (mass%) | 55.1 | 61.2 | 62.8 | 67.2 | 72.3 | 45.4 | 40.8 | 46.0 | 41.3 |
| Crystalline celluloselentire excipient (mass%) | 10.5 | 7.7 | 7.8 | 8.4 | 9.0 | 5.7 | 5.1 | 5.8 | 5.2 |
| Corn starch/lactose hydrate (mass ratio) | 1.6 | 2.0 | 2.1 | 2.8 | 3.9 | 0.9 | 0.8 | 1.0 | 0.8 |
| Crystalline cellulose/lactose hydrate (mass ratio) | 0.31 | 0.25 | 0.27 | 0.34 | 0.48 | 0.12 | 0.09 | 0.12 | 0.10 |

### [Table 1-2]

**[Table 1-2]**

| | Example 4 | Comparative Example 7 | Example 5 | Comparative Example 8 | Example 6 | Example 7 | Example 8 | Example 9 |
|---|---|---|---|---|---|---|---|---|
| Bacterial cell (mass%) | 4.2 | 4.2 | 8.4 | 8.4 | 33.7 | 33.7 | 33.7 | 25.3 |
| Lactose hydrate (mass%) | 39.8 | 49.8 | 35.6 | 45.6 | 10.3 | 10.3 | 10.3 | 23.7 |
| Corn starch (mass%) | 40.0 | 40.0 | 40.0 | 40.0 | 40.0 | 40.0 | 40.0 | 40.0 |
| Crystalline cellulose (mass%) | 5.0 | 5.0 | 5.0 | 5.0 | 5.0 | 5.0 | 5.0 | 5.0 |
| Croscarmellose Na (mass%) | 10.0 | 0.0 | 10.0 | 0.0 | 0.0 | 0.0 | 10.0 | 5.0 |
| Crospovidone (mass%) | 0.0 | 0.0 | 0.0 | 0.0 | 10.0 | 0.0 | 0.0 | 0.0 |
| Na starch glycolate (mass%) | 0.0 | 0.0 | 0.0 | 0.0 | 0.0 | 10.0 | 0.0 | 0.0 |
| Magnesium stearate (mass%) | 1.0 | 1.0 | 1.0 | 1.0 | 1.0 | 1.0 | 1.0 | 1.0 |
| Total | 100.0 | 100.0 | 100.0 | 100.0 | 100.0 | 100.0 | 100.0 | 100.0 |
| Lactose hydrate/entire excipient (mass%) | 46.9 | 52.5 | 44.2 | 50.3 | 18.7 | 18.7 | 18.7 | 34.5 |
| Corn starch/entire excipient (mass%) | 47.2 | 42.2 | 49.6 | 44.2 | 72.3 | 72.3 | 72.3 | 58.2 |
| Crystalline cellulose/entire excipient (mass%) | 5.9 | 5.3 | 6.2 | 5.5 | 9.0 | 9.0 | 9.0 | 7.3 |
| Corn starch/lactose hydrate (mass ratio) | 1.0 | 0.8 | 1.1 | 0.9 | 3.9 | 3.9 | 3.9 | 1.7 |
| Crystalline cellulose/lactose hydrate (mass ratio) | 0.13 | 0.10 | 0.14 | 0.11 | 0.48 | 0.48 | 0.48 | 0.21 |

**[Table 1-3]**

| (Table 1 continued) | | | |
|---|---|---|---|
| | Example 10 | Comparative Example 9 | Example 11 |
| Bacterial cell (mass%) | 29.5 | 33.7 | 33.7 |
| Lactose hydrate (mass%) | 19.5 | 15.3 | 6.3 |
| Corn starch (mass%) | 40.0 | 40.0 | 40.0 |
| Crystalline cellulose (mass%) | 5.0 | 5.0 | 5.0 |
| Croscarmellose Na (mass%) | 5.0 | 5.0 | 14.0 |
| Crospovidone (mass%) | 0.0 | 0.0 | 0.0 |
| Na starch glycolate (mass%) | 0.0 | 0.0 | 0.0 |
| Magnesium stearate (mass%) | 1.0 | 1.0 | 1.0 |
| Total | 100.0 | 100.0 | 100.0 |
| Lactose hydrate/entire excipient (mass%) | 30.3 | 25.4 | 12.3 |
| Corn starch/entire excipient (mass%) | 62.0 | 66.3 | 77.9 |
| Crystalline cellulose/entire excipient (mass%) | 7.7 | 8.3 | 9.7 |
| Corn starch/lactose hydrate (mass ratio) | 2.0 | 2.6 | 6.3 |
| Crystalline cellulose/lactose hydrate (mass ratio) | 0.26 | 0.33 | 0.79 |

### <Disintegration test>

The disintegration test was performed using a disintegration tester (NT-610, TOYAMA SANGYO CO., LTD., Osaka) according to the disintegration test method of encapsulated formulations in "6.09 Disintegration Test Method" in The Japanese Pharmacopoeia 18th edition. Purified water was used for the test, and the test conditions were a temperature of 37 ± 2°C and no auxiliary disk. The disintegration time was the time required for the encapsulated formulation or its contents to completely disappear from a stainless steel mesh of the tester. The test was performed once for each sample, and the average value of disintegration times of six encapsulated formulations was calculated.

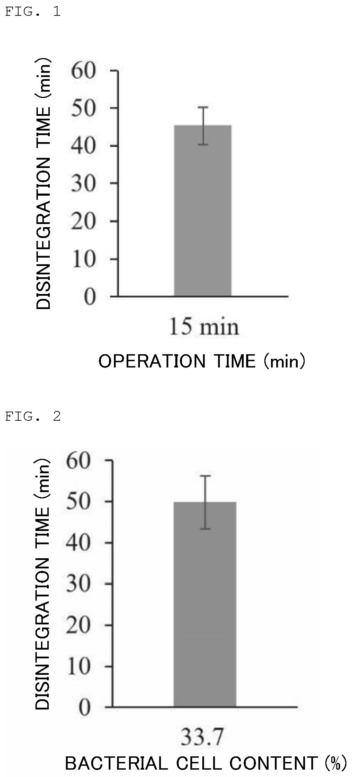

Fig. 1 shows the test results of an encapsulated formulation of Comparative Example 1 (operation time: 15 minutes), and Fig. 2 shows the test results of an encapsulated formulation of Comparative Example 2 (operation time: 0 minutes). Table 2 shows the test results of encapsulated formulations of Examples 1 to 3 (operation time: 0, 5, or 15 minutes). The operation time indicates a time zone in which the encapsulated formulation is produced. The operation time of 0 minutes indicates that the encapsulated formulation is produced between 0 seconds to 59 seconds after the start of production, the operation time of 5 minutes indicates that the encapsulated formulation is produced between 4 minutes 0 seconds and 4 minutes 59 seconds after the start of production, and the operation time of 15 minutes indicates that the encapsulated formulation is produced between 15 minutes 0 seconds and 15 minutes 59 seconds after the start of production.

As shown in Figs. 1 and 2, it can be seen that the encapsulated formulations of Comparative Examples 1 and 2 exceed the standard value (20 minutes) according to the Japanese Pharmacopoeia.

On the other hand, as shown in Table 2, it can be seen that the disintegratability of the encapsulated formulations of Examples 1 to 3 was improved by containing 10 mass% of a disintegrant (croscarmellose sodium).

### [Table 2]

**(Table 2)**

| | | Example 1 | | | Example 2 | | | Example 3 | | |
|---|---|---|---|---|---|---|---|---|---|---|
| Operation time (min) | | 0 | 5 | 10 | 0 | 5 | 10 | 0 | 5 | 10 |
| Disintegration time (min)^{*1} | Minimum | 5.8 | 6.1 | 6.1 | 5.9 | 5.9 | 5.8 | 5.4 | 6.2 | 4.9 |
| | Maximum | 9.7 | 8.8 | 9.2 | 8.3 | 8.2 | 8.3 | 7.4 | 9.1 | 8.6 |

| | | | | | | | | | | |
|---|---|---|---|---|---|---|---|---|---|---|
| *1: Minimum and maximum times among disintegration times of six encapsulated formulations are recorded. | | | | | | | | | | |

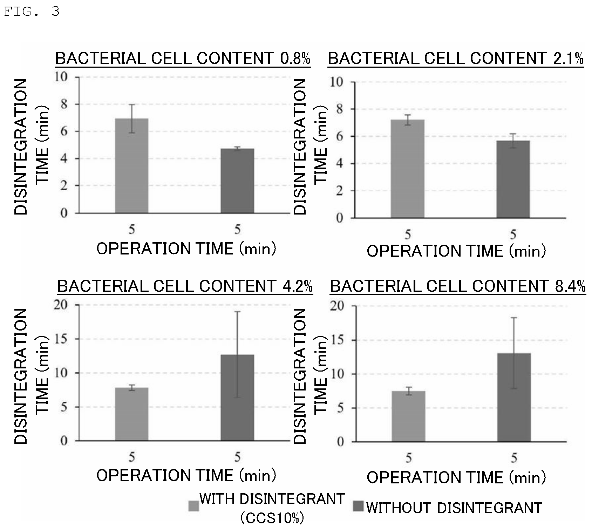

Fig. 3 shows the test results of encapsulated formulations of Examples 4 and 5 and Comparative Examples 3 to 8 (operation time: 5 minutes).

As shown in Fig. 3, it can be seen that in the encapsulated formulations (bacterial cell content: 0.8 mass% or 2.1 mass%) of Comparative Examples 3 to 6, the disintegration time was prolonged by adding 10 mass% of the disintegrant (croscarmellose sodium)

In the encapsulated formulations of Examples 4 and 5 and Comparative Examples 7 and 8 (bacterial cell content: 4.2 mass% or 8.4 mass%), it can be seen that disintegratability was improved when Examples 4 and 5 contained 10 mass% of the disintegrant (croscarmellose sodium).

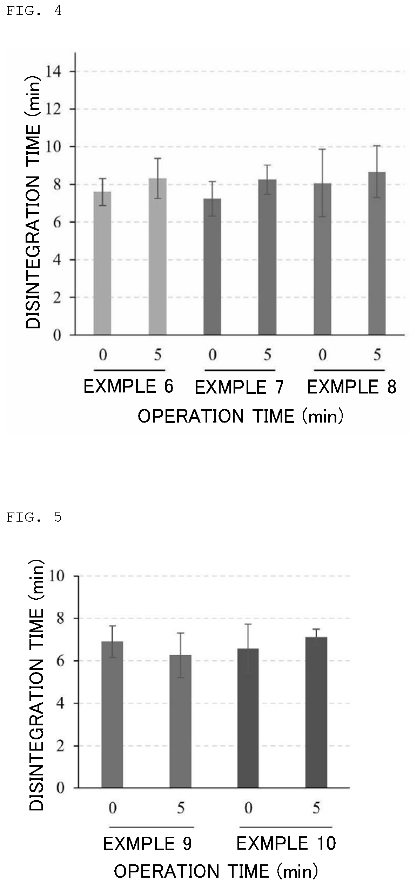

Fig. 4 shows the test results of encapsulated formulations of Examples 6 to 8 (operation time: 0 or 5 minutes). The encapsulated formulation of Example 6 contains crospovidone as a disintegrant, the encapsulated formulation of Example 7 contains sodium starch glycolate as a disintegrant, and the encapsulated formulation of Example 8 contains croscarmellose sodium as a disintegrant.

As shown in Fig. 4, it can be seen that crospovidone and sodium starch glycolate can improve disintegratability similar to croscarmellose sodium.

Fig. 5 shows the test results of encapsulated formulations of Examples 9 and 10 (operation time: 0 or 5 minutes).

As shown in Fig. 5, it can be seen that when the content of the bacterial cell was 25.3 mass% (Example 9) or 29.5 mass% (Example 10), disintegratability was improved by containing 5 mass% of the disintegrant (croscarmellose sodium).

Table 3 shows the test results of encapsulated formulations of Examples 3 and 11 and Comparative Example 9 ((operation time: 0, 5, 10, or 15 minutes (Example 3 and Comparative Example 9), operation time: 0, 5, or 10 minutes (Example 11)).

As shown in Table 3, it can be seen that when the content of the bacterial cell is 33.7 mass%, addition of 5 mass% of the disintegrant (croscarmellose sodium) does not sufficiently improve disintegratability (Comparative Example 9). On the other hand, it can be seen that disintegratability was improved by adding 10 mass% (Example 3) and 14 mass% (Example 11) of the disintegrant (croscarmellose sodium).

### [Table 3]

**(Table 3)**

| | | Comparative Example 9 | | | | Example 3 | | | | Example 11 | | |
|---|---|---|---|---|---|---|---|---|---|---|---|---|
| Operation time (min) | | 0 | 5 | 10 | 15 | 0 | 5 | 10 | 15 | 0 | 5 | 10 |
| Disintegration time (min) | Minimum | 14.8 | 15.2 | 20 | 23.6 | 5.9 | 6.9 | 6 | 5.7 | 6.8 | 6 | 6.6 |
| | Maximum | 60 | >20 | 26.5 | >25.2 | 11.1 | 10.7 | 8.1 | 9.3 | 9.4 | 9.8 | 8.8 |

### <Measurement of filling amount>

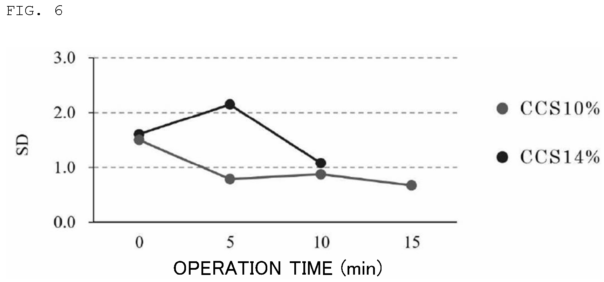

For ten encapsulated formulations (Examples 3 and 11), the mass of the contents of each encapsulated formulation (operation time: 0, 5, 10, or 15 minutes) was measured using an electronic balance (GH-252, A&D Company, Limited, Tokyo), and the average value and the standard deviation were calculated. The results are shown in Fig. 6.

As shown in Fig. 6, it can be seen that the encapsulated formulations of Examples 3 and 11 using the formulation composition can suppress variation in the filling amount.

## Claims

1. A formulation composition comprising:
a bacterial cell;
at least one disintegrant selected from the group consisting of croscarmellose sodium, crospovidone, and sodium starch glycolate; and
an excipient (provided that, croscarmellose sodium, crospovidone, and sodium starch glycolate are excluded), wherein
a content of the bacterial cell is 3.0 mass% or more and 35.0 mass% or less with respect to a total mass of the formulation composition,
when the content of the bacterial cell is 3.0 mass% or more and 29.5 mass% or less with respect to the total mass of the formulation composition, a content of the disintegrant is 5.0 mass% or more and 14.0 mass% or less with respect to the total mass of the formulation composition, and
when the content of the bacterial cell is more than 29.5 mass% and 35.0 mass% or less with respect to the total mass of the formulation composition, the content of the disintegrant is 10.0 mass% or more and 14.0 mass% or less with respect to the total mass of the formulation composition.

2. The formulation composition according to claim 1, wherein the bacterial cell is a bacterial cell of at least one bacteria selected from the group consisting of butyric acid bacteria, lactic acid bacteria, and bifidobacteria.

3. The formulation composition according to claim 1, wherein the bacterial cell is in a form of a dried product prepared by spray drying or a fluidized bed granulation method.

4. The formulation composition according to claim 1, wherein a content of the excipient is 50.0 mass% or more and 85.0 mass% or less with respect to the total mass of the formulation composition.

5. The formulation composition according to claim 1, wherein the excipient contains at least one of lactose hydrate and mannitol, and corn starch.

6. The formulation composition according to claim 5, wherein a content of the at least one of lactose hydrate and mannitol is 12.0 mass% or more and 47.0 mass% or less with respect to the total mass of the excipient.

7. The formulation composition according to claim 5, wherein a mass ratio of the corn starch to the at least one of lactose hydrate and mannitol is 1.0 or more and 6.4 or less.

8. The formulation composition according to claim 5, wherein the excipient further contains crystalline cellulose.

9. The formulation composition according to claim 8, wherein a mass ratio of the crystalline cellulose to the at least one of lactose hydrate and mannitol is 0.12 or more and 0.80 or less.

10. The formulation composition according to claim 1, further comprising a lubricant.

11. An encapsulated formulation comprising the formulation composition according to any one of claims 1 to 10.
